(19) Europäisches Patentamt
European Patent Office
Office européen des brevets

(11) Veröffentlichungsnummer: **0 317 882 B1**

(12) # EUROPÄISCHE PATENTSCHRIFT

(45) Veröffentlichungstag der Patentschrift: **28.07.93**

(51) Int. Cl.⁵: **C07C 217/78**, C07C 205/35, C08G 69/40, C08G 73/10, C08G 73/20

(21) Anmeldenummer: **88119069.8**

(22) Anmeldetag: **17.11.88**

(54) **Fluorhaltige Verbindungen auf Basis von 4,4'-Bis [2-(4-hydroxyphenyl) hexafluorisopropyl] diphenylether, Verfahren zu ihrer Herstellung und ihre Verwendung.**

(30) Priorität: **24.11.87 DE 3739798**
**24.11.87 DE 3739799**

(43) Veröffentlichungstag der Anmeldung:
**31.05.89 Patentblatt 89/22**

(45) Bekanntmachung des Hinweises auf die Patenterteilung:
**28.07.93 Patentblatt 93/30**

(84) Benannte Vertragsstaaten:
**BE CH DE FR GB IT LI NL**

(56) Entgegenhaltungen:
**EP-A- 0 135 647**
**EP-A- 0 192 480**
**EP-A- 0 317 942**
**EP-A- 0 317 944**
**US-A- 4 111 906**
**US-A- 4 521 623**

(73) Patentinhaber: **HOECHST AKTIENGESELLSCHAFT**
**Postfach 80 03 20**
**W-6230 Frankfurt am Main 80(DE)**

(72) Erfinder: **Lau, Jürgen, Dr.**
**Thüringer Weg 2**
**W-6238 Hofheim am Taunus(DE)**
Erfinder: **Siegemund, Günter, Dr.**
**Frankfurter Strasse 21**
**W-6238 Hofheim am Taunus(DE)**

EP 0 317 882 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf neue fluorhaltige Verbindungen auf Basis von 4,4'-Bis[2-(4-hydroxyphenyl)-hexafluorisopropyl]diphenylether, Verfahren zu ihrer Herstellung und ihre Verwendung, wobei die Verbindungen zwei Hexafluorisopropylidengruppen aufweisen.

Fluorhaltige Verbindungen, speziell fluorhaltige Diaminoverbindungen auf Basis von 2,2-Bis[3- bzw. 4-Aminophenoxy)phenyl]hexafluorpropan sind bekannt (US-A 4,111,906 bzw. EP-A-0 192 480). Ebenfalls sind Verbindungen bekannt, die in 2-Stellung am Phenoxykern ein Halogenatom aufweisen (US-A 4,521,623). Diese Verbindungen, die eine einzige Hexafluorisopropylidengruppe aufweisen, werden zur Herstellung von Polyimiden und Polyamiden mit günstigen Eigenschaften wie thermische Stabilität, Widerstand gegen Bestrahlung und mechanische Festigkeit eingesetzt. Es war daher überraschend, daß aus Verbindungen, die zwei Hexafluorisopropylidengruppen im Molekül tragen, Polymerisate hergestellt werden können, die verbesserte Eigenschaften aufweisen.

Gegenstand der Erfindung sind neue Verbindungen auf der Basis von 4,4'-Bis[2-(4-hydroxyphenyl)-hexafluorisopropyl]diphenylether der Formel

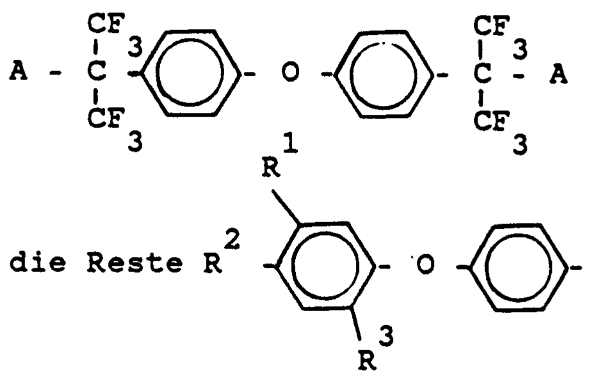

(I)

in der A die Reste

oder

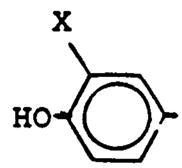

darstellen,

worin $R^1$ und $R^2$ verschieden voneinander sind und Wasserstoff, $-NO_2$ oder $-NH_2$ bedeuten, $R^3$ Wasserstoff oder Halogen ist, X $-NO_2$ oder $NH_2$ darstellt, mit der Maßgabe, daß $R^1$ Wasserstoff ist, wenn $R^2$ $-NO_2$ oder $-NH_2$ ist und $R^2$ Wasserstoff ist, wenn $R^1$ $-NO_2$ oder $-NH_2$ ist.

Gegenstand der Erfindung ist auch ein Verfahren zur Herstellung der Verbindungen der Formel (I) und die Verwendung der daraus erhaltenen Diaminoverbindungen.

Als Ausgangsmaterial für die neuen, fluorhaltigen Verbindungen der Formel (I) dient der 4,4'-Bis-[2-(4-hydroxyphenyl)-hexafluorisopropyl]-diphenylether, der mit aromatischen, gegebenenfalls halogenhaltigen, Nitroverbindungen oder durch Nitrierung zu den entsprechenden Dinitroverbindungen umgesetzt wird. Chlor ist als Halogen bevorzugt. Als aromatische Nitroverbindungen werden vorzugsweise 4-Chlornitrobenzol, 3,4-Dichlornitrobenzol und 1,3-Dinitrobenzol eingesetzt. Das Verhältnis von Diphenylether zu den aromatischen Nitroverbindungen beträgt im allgemeinen 1: (2 bis 3), vorzugsweise 1:(2,1 bis 2,4), bezogen auf molare Mengen. Die eingesetzte 4-Hydroxyphenylverbindung ist nach einem Verfahren erhältlich, das in der am selben Tage eingereichten Patentanmeldung, entsprechend der deutschen Patentanmeldung P 37 39 795.8, Titel: "Teilfluorierte Diphenylether, Verfahren zu ihrer Herstellung und ihre Verwendung" beschrieben worden ist.

Die Umsetzung mit den halogenhaltigen Nitroverbindungen wird in organischen Lösungsmitteln durchgeführt, und zwar in dipolaren aprotischen Lösungsmitteln wie Dimethylformamid, Dimethylacetamid, Dimethylsulfoxid, Dimethylsulfon, Sulfolan, N-Methyl-pyrrolidon-(2).

Mindestens stöchiometrische Mengen einer basischen Verbindung, z.B. Alkalihydroxide wie Natrium- oder Kaliumhydroxid, Ammoniumhydroxid, Alkalihydride, Alkali-oder Erdalkalicarbonate wie Natrium- oder Kaliumcarbonat oder -hydrogencarbonate sowie Alkalialkoholate oder Gemische werden bei der Umsetzung eingesetzt. Die Molverhältnisse betragen im allgemeinen 1: (2 bis 4), vorzugsweise 1: (2,1 bis 3), bezogen auf den Diphenylether.

Die Reaktion wird im allgemeinen bei einer Temperatur von 50 bis 200 °C, vorzugsweise 100 bis 180 °C durchgeführt.

Die Nitrierung des 4,4'-Bis[2-(4-hydroxyphenyl)-hexafluorisopropyl]-diphenylethers wird nach üblichen Methoden durchgeführt. Hierbei wird die Nitrogruppe selektiv in ortho-Position zur Hydroxygruppe eingefügt.

Für die Durchführung der Nitrierungsreaktion können allgemein übliche Nitriersäuregemische eingesetzt werden, z.B. Salpetersäure mit Schwefelsäure, Salpetersäure mit Eisessig, Salpetersäure mit Essigsäureanhydrid oder Salpetersäure mit Wasser. Im allgemeinen werden hochkonzentrierte, wasserfreie Säuregemische eingesetzt; vorzugsweise eignet sich jedoch 98 %ige Salpetersäure bei der Nitrierungsreaktion. Die Reaktion wird dergestalt durchgeführt, daß das Ausgangsprodukt in einem organischen Lösungsmittel gelöst wird, z.B. Tetrahydrofuran, Dioxan, Eisessig, Ethanol oder Gemische, vorzugsweise aber Diethylether, und bei einer Temperatur von -10 bis +50 °C, vorzugsweise 5-15 °C langsam mit der Nitriersäure-Mischung versetzt wird. Die Aufarbeitung erfolgt nach üblichen Methoden.

Die neuen, in erster Stufe erhaltenen Dinitroverbindungen können nach üblichen katalytischen Methoden mit Hydrierkatalysatoren oder nach stöchiometrischen Methoden, z.B. mit Zinn(II)-chlorid-Eisessig, in einer zweiten Reaktionsstufe zu den entsprechenden Diaminoverbindungen reduziert werden. Im allgemeinen werden die isolierten Nitroverbindungen hydriert, jedoch kann nach der stöchiometrischen Methode die Hydrierung auch ohne Isolierung der Verbindung durchgeführt werden.

Als Katalysatoren für die katalytische Reduktion können beispielsweise Platinmetalle, Kupfer, Eisen, Kobalt, Nickel, Mischungen daraus oder Metalloxide bei atmosphärischem oder erhöhtem Druck eingesetzt werden. Palladium wird bevorzugt. Die Katalysatoren können als Metall selbst oder in feinverteilter Form auf Oberflächen wie Kohle, Bariumsulfat, Silikagel, Aluminium und Zeolith verwendet werden. Möglich ist auch die Verwendung von Raney-Nickel. Die Reduktion findet in organischen Lösungsmitteln, z.B. Alkoholen wie Methanol, Ethanol und Isopropylalkohol, Glykolen wie Ethylenglykol und Propylenglykol, Ethern die Diethylether, Dioxan, Tetrahydrofuran und Ethylenglykolmono-methyl- bzw. -ethylether, aliphatischen Kohlenwasserstoffen wie Hexan und Cyclohexan, aromatischen Kohlenwasserstoffen wie Benzol, Toluol und Xylol, Estern wie Ethylacetat und Butylacetat, halogenierten Kohlenwasserstoffen sowie Dimethylformamid und Dimethylsulfoxid statt. Die Temperaturen bei dieser Reaktion liegen im allgemeinen zwischen 10 und 130 °C, vorzugsweise zwischen 20 bis 80 °C, insbesondere zwischen 20 und 60 °C.

Die in zweiter Stufe erhaltenen Diaminoverbindungen eignen sich zur Herstellung von hochtemperaturbeständigen Polykondensaten. Durch Umsetzung mit Tetracarbonsäuren oder deren Derivaten werden Polyimide erhalten, die z.B. niedrige Dielektrizitätskonstanten aufweisen. Andererseits ergibt die Reaktion mit Dicarbonsäurechloriden Polyamide. Weiterhin können neue Monomere und Oligomere erhalten werden, beispielsweise durch Reaktion mit Dianhydriden. Die erhaltenen Imidmonomeren und Oligomeren können durch Additionsreaktionen gehärtet werden. Die erfindungsgemäßen Diamide sind weiterhin geeignet zur Herstellung von polymeren Vorprodukten, Epoxidharzhärtern, Matrixharzen, Laminaten, Filmen, Fasern, Klebstoffen, Beschichtungen, Fotoresists und Formkörpern.

Es ist bekannt, Polyamide mit außergewöhnlichen physikalischen und chemischen Eigenschaften herzustellen aus einem organischen Diamin, das ein aromatisches tetravalentes Radikal darstellt und bei dem jede Aminogruppe direkt an einem Kohlenstoffatom eines aromatischen Ringes sitzt, wobei in ortho- oder peri-Stellung zu diesem Kohlenstoffatom eine OH-Gruppe sitzen kann, und das mit einer halogensubstituierten Dicarbonsäure, einem Anhydrid, niederen Alkylestern oder Vinylestern dieser Carbonsäure umgesetzt wird (US-A 3,449,296). Aus den sich ergebenden polymeren Hydroxyamiden können durch übliche Methoden Formkörper hergestellt werden; sie können aber auch durch Hitzebehandlung unter Abspaltung von Wasser in die entsprechenden Polybenzoxazole übergeführt werden. Der erfindungsgemäße 4,4'-Bis[2-(3-amino-4-hydroxyphenyl)hexafluorisopropyl]diphenylether kann ebenfalls als Monomeres für derartige Polyamide und Polybenzoxazole dienen.

## Beispiele

1) 4,4'-Bis[ 2-(4-(4-nitrophenoxy)phenyl)hexafluorisopropyl]diphenylether

13,1 g 4,4'-Bis[2-(4-hydroxyphenyl)hexafluorisopropyl]diphenylether, 50 ml Dimethylacetamid, 20 ml Toluol, 1,8 g NaOH und 3 ml Wasser wurden solange unter Rückfluß erhitzt, bis kein Wasser mehr am Wasserabscheider abgeschieden wurde. Anschließend wurde das Toluol abdestilliert und die Reaktionsmischung nach der Zugabe von 7,1 g 4-Chlornitrobenzol 24 Stunden unter Rückfluß erhitzt. Nach dem Erkalten der Reaktionsmischung auf 25 °C wurde der Feststoffanteil abfiltriert und verworfen. Aus dem Filtrat wurde das Dimethylacetamid destillativ unter vermindertem Druck abgetrennt und der Rückstand aus Acetonitril umkristallisiert. Es verblieben 12,5 g (71 % d.Th.) eines beigefarbenen Feststoffes, der bei 160 °C schmolz.

Die kernresonanzspektroskopische Analyse ergab folgende Werte:
$^{1}$H-NMR ($CDCl_3$)δ(ppm) : 8,2 - 8,3 m 4H, 7,15 - 7,5 m 8H, 7,0 - 7,1 m 12H;
$^{19}$F-NMR ($CDCl_3$)δ(ppm): -64,4 s

| Analyse für $C_{24}H_{24}F_{12}N_2O_7$: | | | | | |
|---|---|---|---|---|---|
| ber.: | C 56,26, | H 2,70, | F 25,43, | N 3,13, | O 12,49 |
| gef.: | C 55,70, | H 2,60, | F 25,30, | N 2,90, | O 12,40 |

2) 4,4′-Bis[2-(4-(4-aminophenyl)phenyl)hexafluorisopropyl]diphenylether

19 g 4,4′-Bis[2-(4-(4-nitrophenoxy)phenyl)hexafluorisopropyl]diphenylether wurden in 250 ml Essigsäureethylester gelöst und unter Zusatz von 1 g Palladium auf Kohle (5 % Pd-Anteil) in einem 1 l Autoklaven mit Wasserstoff (100 bar) bei 25-30 °C reduziert. Nach der Reduktion wurde der Katalysator abfiltriert und das Lösungsmittel abdestilliert. Das Rohprodukt (18,1 g) wurde in der Wärme in einer Lösung aus Oktan/Toluol (19/1) gelöst und mit 3 g Aktivkohle behandelt. Die blaßgelbe Lösung wurde nochmals vollständig eingeengt und der Rückstand aus Toluol umkristallisiert. Ausbeute: 15,4 g (85 % d.Th.) blaßgelber Feststoff, Fp.: 91-93 °C.

Kernresonanzspektroskopie:
$^{1}$H-NMR ($CDCl_3$)δ(ppm) : 7,4 - 6,6 m 24H, 3,5 breites s 4H
$^{19}$F-NMR ($CDCl_3$)δ(ppm): -64,6 s

| Analyse für $C_{42}H_{28}F_{12}N_2O_3$: | | | | | |
|---|---|---|---|---|---|
| ber.: | C 60,29, | H 3,37, | F 27,25, | N 3,35, | O 5,74 |
| gef.: | C 60,20, | H 3,20, | F 26,80, | N 3,50, | O 5,80 |

3) 4,4′-Bis[2-(4-(3-nitrophenoxy)phenyl)hexafluorisopropyl]diphenylether

196 g 4,4′-Bis[2-(4-hydroxyphenyl)-hexafluorisopropyl]diphenylether wurden in 1000 ml Dimethylformamid gelöst und nach der Zugabe von 120 g 1,3-Dinitrobenzol sowie 114 g Kaliumcarbonat 8 Stunden unter Rückfluß erhitzt. Die erkaltete Reaktionsmischung wurde in 10 l Wasser eingetragen und nach der Zugabe von 10 ml konzentrierter Salzsäure filtriert. Der abgetrennte Feststoff wurde intensiv mit Wasser gewaschen. Ausbeute: 217 g (81 % d.Th.) hellbrauner Feststoff, Fp.: 105-107 °C

Kernresonanzspektroskopie:
$^{1}$H-NMR ($CDCl_3$)δ(ppm) : 7,0 - 7,1 m 8H, 7,3 - 7,5 m 12H, 7,9 - 8,0 m 4H
$^{19}$F-NMR ($CDCl_3$)δ(ppm) : -64,47 s

4) 4,4′-Bis[2-(4-(3-aminophenoxy)phenyl)hexafluorisopropyl]diphenylether

89 g 4,4′-Bis[2-(4-(3-nitrophenoxy)phenyl)hexafluorisopropyl]diphenylether wurden in 3000 ml Ethanol gelöst und in einem Stahlautoklaven nach der Zugabe von 2 g Palladiumkohle (5 %ig) bei 50 °C mit Wasserstoff (100 bar) reduziert. Nach dem Abfiltrieren des Katalysators wurde das Lösungsmittel vollständig abdestilliert. Das Rohprodukt wurde in Methanol gelöst und in der Siedehitze mehrmals mit Aktivkohle behandelt.
Ausbeute: 54 g (64 % d.Th.) blaßgelber Feststoff, Fp.: 74-77 °C

Kernresonanzspektroskopie:
$^{1}$H-NMR ($CDCl_3$)δ(ppm) : 3,6 breites s 4H, 6,4 - 6,5 m 6H, 6,9 - 7,1 m 12H, 7,2 - 7,4 m 6H
$^{19}$F-NMR ($CDCl_3$)δ(ppm): -64,5 s

| Analyse für $C_{42}H_{28}F_{12}N_2O_3$: | | | | | |
|---|---|---|---|---|---|
| ber.:<br>gef.: | C 60,29,<br>C 60,70, | H 3,27,<br>H 3,30, | F 27,25,<br>F 26,80, | N 3,35,<br>N 3,60, | O 5,74<br>O 6,20 |

5) 4,4'-Bis [2-(4-(2-chloro-4-nitrophenoxy)phenyl)hexafluorisopropyl]diphenylether

39,2 g 4,4'-Bis[2-(4-hydroxyphenyl)hexafluorisopropyl]diphenylether wurden in 250 ml Dimethylformamid gelöst und nach der Zugabe von 25 g 3,4-Dichlornitrobenzol sowie 22 g Kaliumcarbonat 4 Stunden unter Rückfluß erhitzt. Die erkaltete Reaktionsmischung wurde in 1000 ml Wasser getropft und nach der Zugabe von 10 ml konz. Salzsäure filtriert. Der abgetrennte Feststoff wurde mit Wasser gewaschen und nach dem Trocknen aus Acetonitril umkristallisiert.
Ausbeute: 37 g (64 % d.Th.), Fp.: 154 - 156 °C

Kernresonanzspektroskopie:
$^1$H-NMR ($CDCl_3$)δ(ppm): 7,0 - 7,1 m 1OH, 7,4 - 7,5 m 8H, 8,1 - 8,2 2d 2H, 8,4 d 2H,
$^{19}$F-NMR ($CDCl_3$)δ(ppm): -64,5 s

6) 4,4'-Bis[2- (4- (4- amino-2-chlorphenoxy)phenyl)hexafluorisopropyl]diphenylether

29 g 4,4'-Bis[2-(4-(2-chloro-4-nitrophenoxy)-phenyl)hexafluorisopropyl]diphenylether wurden in 300 ml Essigester (Essigsäureäthylester) gelöst und nach der Zugabe von 1 g Palladiumkohle (5 %ig) in einem Stahlautoklaven mit Wasserstoff (100 bar) bei 50 °C reduziert. Nach der Abtrennung des Katalysators wurde der Essigester am Rotationsverdampfer vollständig abgetrennt. Das Rohprodukt wurde in 30 ml verdünnte Salzsäure eingetragen und aus Ethanol umkristallisiert, wobei die Lösung in der Siedehitze mit Aktivkohle behandelt wurde. Nach dem Abkühlen der Reaktionsmischung auf +10 °C wurde der ausgefallene weiße Niederschlag abfiltriert, mit Wasser gewaschen und in 400 ml Wasser suspendiert. Die Suspension wurde mit verdünnter Ammoniak-Lösung neutralisiert, der Feststoff anschließend abfiltriert und mit Wasser gewaschen.
Ausbeute: 21 g (72 % d.Th.) weißer Feststoff, Fp.: 93-95 °C

Kernresonanzspektroskopie:
$^1$H-NMR ($CDCl_3$)δ(ppm): 3,5 breites s 4H, 6,5 - 6,6 2d 2H, 6,8 - 7,4 m 20H
$^{19}$F-NMR ($CDCl_3$ )δ(ppm): -64,5 s

7) 4,4'-Bis[2-(4-hydroxy-3-nitrophenyl)hexafluorisopropyl]diphenylether

131 g 4,4'-Bis[2-(4-hydroxyphenyl)hexafluorisopropyl]diphenylether wurden in 700 ml Diethylether gelöst und bei 10 °C tropfenweise mit 76 ml konzentrierter Salpetersäure versetzt. Die Reaktionsmischung wurde 1 Stunde gerührt und anschließend auf 300 ml Wasser-Eismischung gegossen. Die organische Phase wurde abgetrennt, mit kaltgesättigter Natriumhydrogenkarbonat-Lösung neutral gewaschen, einmal mit Wasser gewaschen und über Magnesiumsulfat getrocknet. Nach dem Abtrennen des Lösungsmittels verblieben 123 g (83 % d.Th.) eines blaßgelben Feststoffes. Fp.: 67 - 70 °C.

Die Kernresonanzspektroskopie ergab folgende Werte:
$^1$H-NMR ($CDCl_3$)δ(ppm): 7-7,75 m 12H, 8,25 d 2H, 10,7 2H
$^{19}$F-NMR ($CDCl_3$)δ(ppm): -64,6 s

8) 4,4'-Bis [3-amino-4-hydroxyphenyl)hexafluorisopropyl]diphenylether

150 g 4,4'-Bis[2-(4-hydroxy-3-nitrophenyl)hexafluorisopropyl]diphenylether wurden in 1000 ml Ethanol gelöst und unter Zusatz von 4 g Palladiumkohle (10 %ig an Pd) in einem 2 l Autoklaven mit Wasserstoff (100 bar) bei 25 °C reduziert. Nach der Reduktion wurde der Katalysator abfiltriert und das Lösungsmittel am Rotationsverdampfer abdestilliert. Das Rohprodukt wurde zur Reinigung zweimal aus Toluol umkristallisiert.
Ausbeute: 82 g (60 % d.Th. ) weißgrauer Feststoff, gaschromatographische Reinheit größer 99,5 %. Fp.: 208 - 209 °C.

| Analyse für $C_{30}H_{20}F_{12}N_2O_3$: | | | | | |
|---|---|---|---|---|---|
| ber.: | C 52,64, | H 2,95, | N 4,09, | O 7,01, | F 33,31, |
| gef.: | C 52,90, | H 3,00, | N 4,10, | O 7,20, | F 33,50. |

Kernresonanzspektroskopie:
$^{1}$H-NMR ($CDCl_3$) δ(ppm): 9.3-10.0 breites s 2H, 7,1-7,5 m 8H, 6,3-6,8 m 6H 4,8 breites s 4H.
$^{19}$F-NMR ($CDCl_3$) δ(ppm): -67, 7 s

## Patentansprüche

1. Verbindungen der Formel

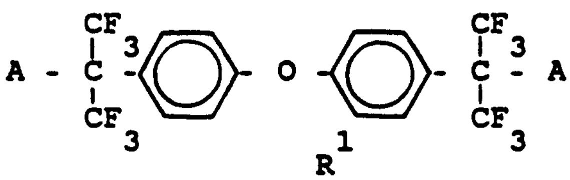

(I)

in der A die Reste 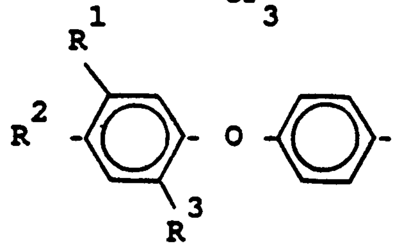 oder 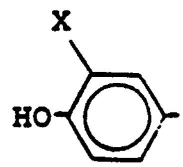

darstellen,
worin $R^1$ und $R^2$ verschieden voneinander sind und Wasserstoff, $-NO_2$ oder $-NH_2$ bedeuten, $R^3$ Wasserstoff oder Halogen ist, X $-NO_2$ oder $NH_2$ darstellt, mit der Maßgabe, daß $R^1$ Wasserstoff ist, wenn $R^2$ $-NO_2$ oder $-NH_2$ ist und $R^2$ Wasserstoff ist, wenn $R^1$ $-NO_2$ oder $-NH_2$ ist.

2. Verbindung nach Anspruch 1, dadurch gekennzeichnet, daß das Halogen Chlor ist.

3. Verbindung der Formel

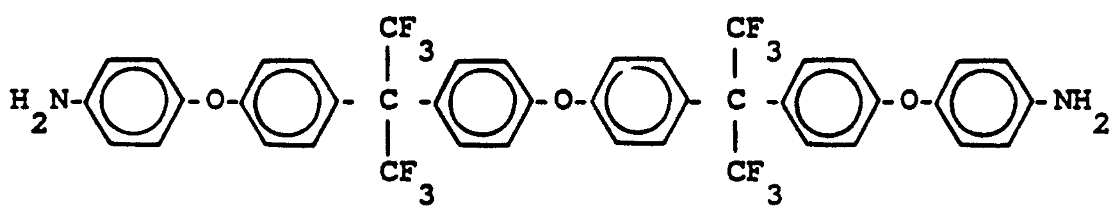

4. Verbindung der Formel

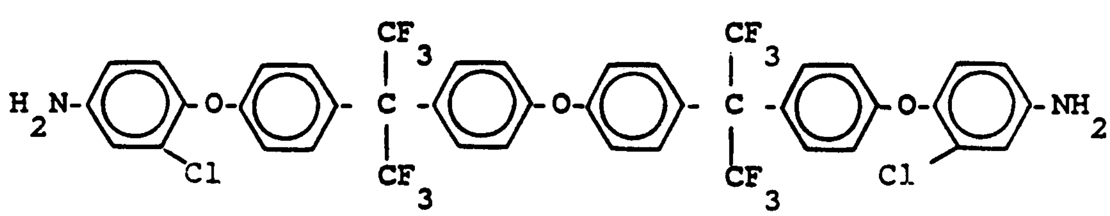

5. Verbindung der Formel

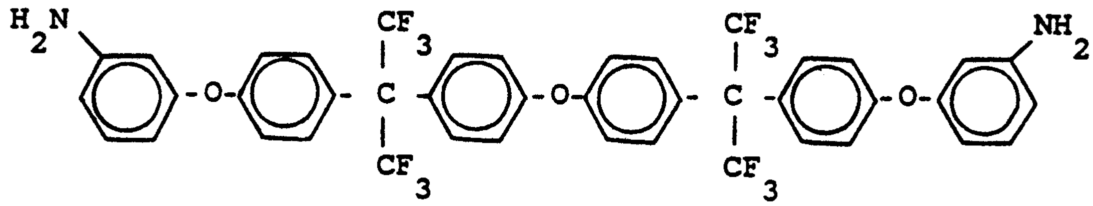

6. 4,4′-Bis[2-(4-hydroxy-3-nitrophenyl)hexafluorisopropyl]diphenylether.

7. 4,4′-Bis[2-(3-amino-4-hydroxyphenyl)hexafluorisopropyl]diphenylether.

8. Verfahren zur Herstellung von Verbindungen gemäß einem oder mehreren der Ansprüche 1 bis 7 a) entweder durch Umsetzung von aromatischen Nitroverbindungen mit einer entsprechenden Dihydroxyverbindung oder b) durch Nitrierung einer entsprechenden Dihydroxyverbindung und c) gegebenenfalls anschließende Reduktion, dadurch gekennzeichnet, daß 4,4′-Bis-[2-(4-hydroxyphenyl)-hexafluorisopropyl]-diphenylether a) in einem dipolaren aprotischen Lösungsmittel gelöst und in Gegenwart von mindestens stöchiometrischen Mengen einer basischen Verbindung mit Halogennitrobenzol, Dihalogennitrobenzol oder Dinitrobenzol bei Temperaturen von 50 bis 200 °C zur Reaktion gebracht oder b) in einem organischen Lösungsmittel gelöst und mit einem Nitriersäuregemisch bei -10° bis +50 °C nitriert wird und c) das Reaktionsprodukt als solches isoliert oder in einer zweiten Stufe I) ohne Isolierung stöchiometrisch hydriert oder II) nach Isolierung katalytisch oder stöchiometrisch hydriert wird.

9. Verfahren nach Anspruch 8, dadurch gekennzeichnet, daß als Nitrobenzol 4-Chlornitrobenzol, 3,4-Dichlornitrobenzol oder 1,3-Dinitrobenzol und daß als aprotische Lösungsmittel vorteilhaft Dimethylformamid, Dimethylacetamid, Dimethylsulfoxid, Dimethylsulfon, Sulfolan oder N-Methyl-pyrrolidon-(2) einzeln oder im Gemisch eingesetzt werden.

10. Verfahren nach Anspruch 8 oder 9, dadurch gekennzeichnet, daß als basische Verbindungen Alkalihydroxide, Ammoniumhydroxid, Alkalihydride, Alkali- oder Erdalkalicarbonate oder -hydrogencarbonate odere Alkalialkoholate und daß als Nitriersäuregemisch vorteilhaft Gemische von Salpetersäure mit Schwefelsäure, Eisessig, Essigsäureanhydrid oder Wasser, vorzugsweise 98 %ige Salpetersäure eingesetzt werden.

11. Verfahren nach Anspruch 8 oder 10, dadurch gekennzeichnet, daß als organische Lösungsmittel Tetrahydrofuran, Dioxan, Eisessig, Ethanol, vorzugsweise Diethylether eingesetzt werden.

12. Verfahren nach einem oder mehreren der Ansprüche 8, 10 oder 11, dadurch gekennzeichnet, daß die Temperatur bei der Nitrierung 5 bis 15 °C beträgt.

13. Verfahren nach einem oder mehreren der Ansprüche 8 bis 12, dadurch gekennzeichnet, daß für die katalytische Hydrierung Metallkatalysatoren aus der Gruppe der Platinmetalle, Kupfer, Eisen, Kobalt, Nickel, Mischungen daraus oder Metalloxide bei Temperaturen von 10 bis 130 °C eingesetzt werden.

14. Verwendung der Diaminoverbindungen gemäß den Ansprüchen 1 bis 5 und 7 zur Herstellung von hochtemperaturbeständigen Polykondensaten.

15. Verwendung nach Anspruch 14 zur Herstellung von Polyimiden und Polyamiden oder Polybenzoxazolen.

## Claims

**1.** A compound of the formula

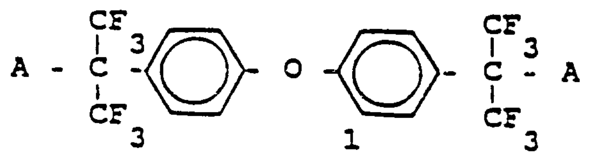

(I)

in which A represents the radicals

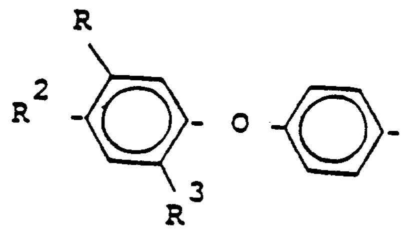

or

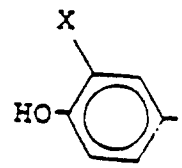

in which $R^1$ and $R^2$ are different from one another and denote hydrogen, $-NO_2$ or $-NH_2$, $R^3$ is hydrogen or halogen, X represents $-NO_2$ or $-NH_2$, with the proviso that $R^1$ is hydrogen, if $R^2$ is $-NO_2$ or $-NH_2$ and $R^2$ is hydrogen, if $R^1$ is $-NO_2$ or $-NH_2$.

**2.** The compound as claimed in claim 1, wherein the halogen is chlorine.

**3.** A compound of the formula

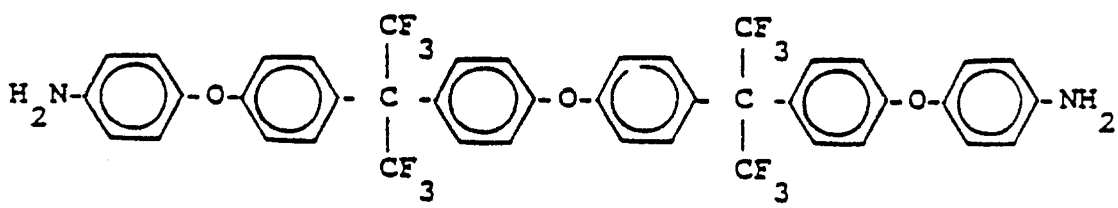

**4.** A compound of the formula

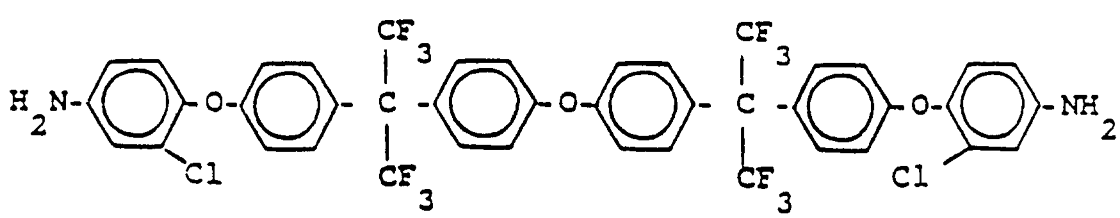

**5.** A compound of the formula

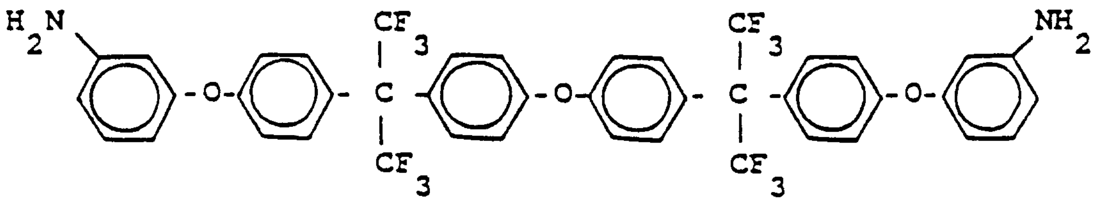

**6.** 4,4'-bis[2-(4-hydroxy-3-nitrophenyl)hexafluoroisopropyl]diphenyl ether.

**7.** 4,4'-bis[2-(3-amino-4-hydroxyphenyl)hexafluoroisopropyl]diphenyl ether.

**8.** A process for the preparation of a compound as claimed in one or more of claims 1 to 7 a) either by reaction of aromatic nitro compounds with an appropriate dihydroxy compound or b) by nitration of an appropriate dihydroxy compound and c) if necessary followed by reduction, which comprises dissolving 4,4'-bis[2-(4-hydroxyphenyl)hexafluoroisopropyl]diphenyl ether a) in a dipolar aprotic solvent and reacting it in the presence of at least stoichiometric amounts of a basic compound with halonitrobenzene, dihalonitrobenzene or dinitrobenzene at temperatures of 50 to 200 °C or b) dissolving it in an organic solvent and nitrated with a nitrating acid mixture at -10 °C to +50 °C and c) isolating the reaction product as such or in a second step I) hydrogenating it stoichiometrically without isolation or II) hydrogenating it after isolation catalytically or stoichiometrically.

**9.** The process as claimed in claim 8, wherein the nitrobenzene used is 4-chloronitrobenzene, 3,4-dichloronitrobenzene or 1,3-dinitrobenzene and the aprotic solvents used are advantageously dimethylformamide, dimethylacetamide, dimethyl sulfoxide, dimethyl sulfone, sulfolane or N-methyl-2-pyrrolidone individually or in the mixture.

**10.** The process as claimed in claim 8 or 9, wherein the basic compounds used are alkali metal hydroxides, ammonium hydroxide, alkali metal hydrides, alkali metal carbonates, alkaline earth metal carbonates, alkali metal bicarbonates, alkaline earth metal bicarbonates or alkali metal alcoholates and the nitrating acid mixture used is advantageously a mixture of nitric acid with sulfuric acid, glacial acetic acid, acetic anhydride or water, preferably 98% strength nitric acid.

**11.** The process as claimed in claim 8 or 10, wherein the organic solvent used is tetrahydrofuran, dioxane, glacial acetic acid, ethanol, preferably diethyl ether.

**12.** The process as claimed in one or more of claims 8, 10 or 11, wherein the temperature of nitration is 5 to 15 °C.

**13.** The process as claimed in one or more of claims 8 to 12, wherein for the catalytic hydrogenation metal catalysts from the group consisting of platinum metals, copper, iron, cobalt, nickel, mixtures thereof or metal oxides are used at temperatures of 10 to 130 °C.

**14.** Use of the diamino compounds as claimed in claims 1 to 5 and 7 for the preparation of high-temperature-resistant polycondensates.

**15.** Use as claimed in claim 14 for the preparation of polyimides and polyamides or polybenzoxazoles.

## Revendications

**1.** Composés de formule :

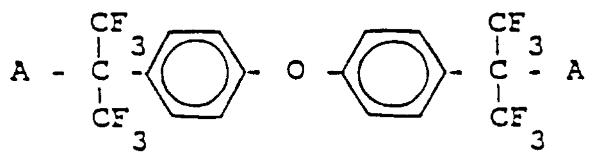

(I)

dans laquelle A représente les radicaux :

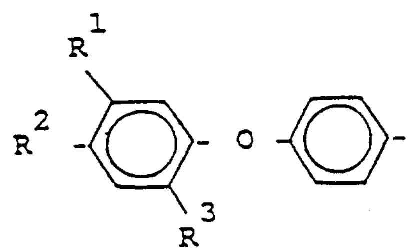

ou

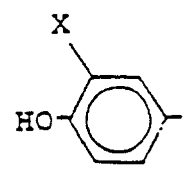

où $R^1$ et $R^2$ sont identiques ou différents et représentent chacun un hydrogène ou le radical $NO_2$ ou $NH_2$, $R^3$ est un hydrogène ou un halogène, X est un radical $NO_2$ ou $NH_2$, du moment que $R^1$ est un hydrogène quand $R^2$ est $NO_2$ ou $NH_2$ et que $R^2$ est un hydrogène quand $R^1$ est $NO_2$ ou $NH_2$.

**2.** Composé selon la revendication 1, caractérisé en ce que l'halogène est le chlore.

**3.** Composé de formule :

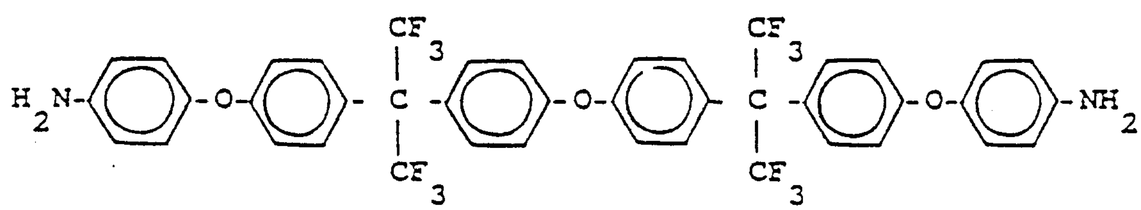

**4.** Composé de formule :

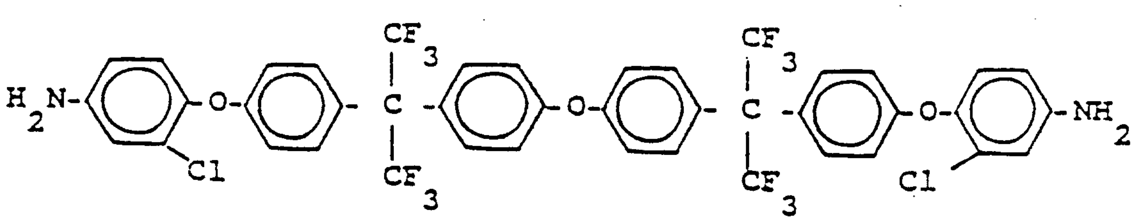

5. Composé de formule :

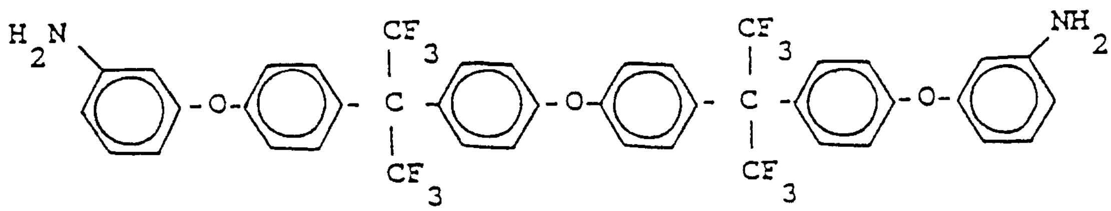

6. Oxyde de bis[(hydroxy-4 nitro-3 phényl)-2 hexafluoroisopropyl]-4,4' diphényle.

7. Oxyde de bis[(amino-3 hydroxy-4 phényl)-2 hexafluoroisopropyl]-4,4' diphényle.

8. Procédé pour préparer des composés selon l'une ou plusieurs des revendications 1 à 7, a) par réaction de composés nitrés aromatiques avec un composé dihydroxylé correspondant, ou b) par nitration d'un composé dihydroxylé correspondant, c) éventuellement suivie d'une réduction, caractérisé en ce que : a) on dissout dans un solvant aprotique dipolaire l'oxyde de bis[(hydroxy-4 phényl)-2 hexafluoroisopropyl]-4,4' diphényle et, en présence de quantités au moins stoechiométriques d'un composé basique, on le fait réagir avec un halogénonitrobenzène, un dihalogénonitrobenzène ou du dinitrobenzène à des températures de 50 à 200 °C, ou bien b) on le dissout dans un solvant organique et on le nitre à une température de -10 à +50 °C avec un mélange d'acides de nitration, et c) on isole le produit de réaction en tant que tel, ou encore, dans une deuxième étape, on lui fait subir (I) une hydrogénation stoechiométrique sans isolement, ou (II) une hydrogénation catalytique ou stoechiométrique après isolement.

9. Procédé selon la revendication 8, caractérisé en ce qu'on utilise comme nitrobenzène le chloro-4 nitrobenzène, le dichloro-3,4 nitrobenzène ou le dinitro-1,3 benzène, et qu'on utilise avantageusement en tant que solvant aprotique le diméthylformamide, le diméthylacétamide, le diméthylsulfoxyde, la diméthylsulfone, le sulfolanne ou la N-méthylpyrrolidone-2, seuls ou en mélanges.

10. Procédé selon la revendication 8 ou 9, caractérisé en ce qu'on utilise comme composés basiques des hydroxydes de métaux alcalins, de l'hydroxyde d'ammonium, des hydrures de métaux alcalins, des carbonates ou hydrogénocarbonates de métaux alcalins ou alcalinoterreux ou des alcoolates de métaux alcalins, et qu'on utilise comme mélange d'acides de nitration avantageusement des mélanges d'acide nitrique avec de l'acide sulfurique, de l'acide acétique glacial, de l'anhydride acétique ou de l'eau, de préférence avec de l'acide nitrique à 98 %.

11. Procédé selon la revendication 8 ou 10, caractérisé en ce qu'on utilise comme solvants organiques le tétrahydrofuranne, le dioxanne, l'acide acétique glacial, l'éthanol, de préférence l'oxyde de diéthyle.

12. Procédé selon l'une ou plusieurs des revendications 8, 10 ou 11, caractérisé en ce que la température lors de la nitration est de 5 à 15 °C.

13. Procédé selon l'une ou plusieurs des revendications 8 à 12, caractérisé en ce qu on utilise pour l'hydrogénation catalytique des catalyseurs métalliques choisis parmi l'ensemble comprenant les métaux du groupe du platine, le cuivre, le fer, le cobalt, le nickel, leurs mélanges ou des oxydes métalliques, à des températures de 10 à 130 °C.

14. Utilisation des composés diaminés selon les revendications 1 à 5 et 7, pour préparer des produits de polycondensation résistant aux hautes températures.

15. Utilisation selon la revendication 14, pour préparer des polyimides et polyamides ou polybenzoxazoles.